# EUROPEAN PATENT APPLICATION

(11) **EP 0 930 294 A1**
(43) Date of publication of application: **21.07.1999**
(21) Application number: 97937826.2
(22) Date of filing: 28.08.1997
(51) Int. Cl.: C07C 237/22, C07C 231/24

(54) **METHOD FOR PURIFYING N-ACYLAMINO ACID AMIDES**

(30) Priority: 28.08.1996 JP 22653196; 30.08.1996 JP 23069796
(71) Applicant: Ajinomoto Co., Inc., Tokyo 104 (JP)
(72) Inventor: TABOHASHI,T., Central Res.Lab. Ajinomoto Co., Inc, Kanagawa-ken 210 (JP); YAMATO, Naoya Central Research Laboratories, Kawasaki-shi Kanagawa-ken 210 (JP); KIYOOKA, Fumiharu Central Research Laboratories, Kawasaki-shi Kanagawa-ken 210 (JP); IKEDA, Toru Central Research Laboratories, Kawasaki-shi Kanagawa-ken 210 (JP)
(74) Representative: Nicholls, Kathryn Margaret
(86) International application number: JP9703005
(87) International publication number: WO9808806

(57) **Abstract**

Disclosed herein are a method of purifying an N-acylamino acid amide comprising adding water and a specific organic solvent to an N-acylamino acid amide, distillating azeotropically the water and the organic acid (with further water being added from time to time if necessary), whereby the N-acylamino acid amide gets precipitated in the form of granular solid excellent in separability as the organic solvent gets solvent-replaced by the water in the course of the azeotropic distillation, and separating the solid, and the method of purifying an N-acylamino acid amide when applied to an N-acylamino acid amide contaminated with impurities such as the alkylamine used as a starting material but remaining unreacted, further comprising subjecting the said N-acylamino acid amide to the acid extraction and/or the alkali extraction, whereby the inpurities are removed.

## Description

### (Technical Field)

The present invention relates to a method of purifying N-acylamino acid amides. N-Acylamino acid amides are known as having a function of solidifying, into a gel state, agar state or block state, organic solvents in the form of a liquid at normal temperature, particularly combustible organic mediums such as natural or synthetic mineral oils, animal oils, vegetable oils etc., and noncombustible organic mediums such as organic phosphorus compounds, organic chloride type compounds etc. (Japanese Patent Publications (kokoku) Nos. 42,079/1976, 13,434/1978 and 27,776/1978). They are accordingly useful in various fields, e.g., as a gelling aget for crude oil spilled from shipwrecked tankers, waste oils from home or the like, and as base materials for cosmetics.

### (Background Art)

As for the synthesis of N-acylamino acid amides is known, e.g., a method (amidation method) wherein an N-acylamino acid employed as one starting material is reacted with an alkylamine as the other starting material. For example, Jap. Pat. Pub. (kokoku) No. 18,691/1977 discloses a method wherein an N-acylamino acid is heated for reaction along with an alkylamine without activating the amino acid, and in greater detail, an N-acylamino acid is added with 1.1-1.2 moles per 1 mole of the N-acylamino acid, of an alkylamine, the resultant mass being a 1 : 1.1-1.2 molar ratio mixture of the N-acylamino acid and the alkylamine, and then heated for reaction in the presence or absence of a solvent such as toluene or xylene. According to the disclosure is purified the resultant crude N-acylamino acid amide product, by removing by evaporation the reaction solvent, heat-dissolving the resultant residue in ethyl acetate, and recrystallizing the N-acylamino acid amide from the solution by cooling. However, recrystallization with the use of, e.g., methanol or ethyl acetate, gives an N-acylamino acid amide in the form of fibrous crystals, which, in turn, deteriorates the separability such as filterability of the N-acylamino acid amide from the remainder and therefore, causes remarkable difficulties in the commercial production of N-acylamino acid amides. Furthermore, impurities such as, e.g., the alkylamine used as a starting material but remaining unreacted, are difficult to remove. These remaining impurities and the decomposition products of the alkylamine are also extremely problematic in that they may give rank odors, coloration etc. to an N-acylamino acid amide product obtained by such method. Thus, there has not been known any commercially satisfactory method of purifying N-acylamino acid amides.

In view of the above-described prior-art background, it is an object of the present invention to provide a method of purifying a crude N-acylamino acid amide product, whereby the N-acylamino acid amide is formed as a solid excellent in separability such as filterability and can, therefore, be commercially easy to separate, in other words, a purifying method, whereby an N-acylamino acid amide product of a higher purity can be obtained which can be commercially used more usefully, as far as the production method of an N-acylamino acid amide wherein an N-acylamino acid and an amine are reacted with each other, is concerned.

### (Disclosure of Invention)

The inventors of the present invention have intensively and extensively studied to achieve the above-mentioned object, and have found that a synthetic reaction mixture of an N-acylamino acid amide containing the N-acylamino acid amide therein or an residue resulting from removing by evaporation the solvent from such synthetic reaction mixture, is added with water and particular organic solvent(s), and the resultant mass is subjected to azeotropic distillation, whereby an N-acylamino acid amide product can be obtained in the form of a solid having good separability such as filterability, and further that acid-extraction and/or alkali-extraction of impurities, when carried out prior to such azeotropic distillation, can give an N-acylamino acid amide product in the solid form and having a higher purity. On the basis of these findings, they have completed the present invention.

Accordingly, the present invention relates to a method of purifying N-acylamino acid amide which comprises distilling off azeotropically the organic solvent together with water, whereby the N-acylamino acid amide is solidified, and separating the solidified N-acylamino acid amide from the remainder, in the steps wherein the N-acylamino acid amide is separated from, e.g., a synthetic reaction mixture resulting from reaction of an N-acylamino acid with an alkylamine and contaminated with impurities such as, e.g., alkylamine used as a starting material but remaining unreacted, and is purified, and also to the above-described method which further comprises acid-extraction and/or alkali-extraction of impurities prior to the azeotropic distillation.

N-acylamino acid amides typically represented by N-acyl acidic amino acid amides are, as has been described earlier, known as having a function of solidifying, into a gel state, agar state or block state, organic solvents in the form of a liquid at normal temperature, particularly combustible organic mediums such as natural or synthetic mineral oils, animal oils, vegetable oils etc., and noncombustible organic mediums such as organic phosphorus compounds, organic chloride type compounds etc.. They are accordingly useful in various fields, e.g., as a gelling aget for crude oil spilled from shipwrecked tankers, waste oils from home or the like, and as base materials for cosmetics. Among N-acylamino acid amides available now on the market is "Oil-gelling Agent GP-1" (N-lauroyl-L-glutamic acid-α, γ-di-n-butylamide, ex Ajinomoto Co., Inc.).

N-acylamino acid amides to be purified according to the method of the present invention can be, as has been described earlier, synthesized by the method disclosed in Jap. Pat. Pub. (kokoku) No. 18,691/1977. They can be also synthesized as follows; an N-acylamino acid such as N-acylglutamic acid is first heated with methyl alcohol under reflux in the presence of sulfuric acid as catalyst, whereby the N-acylamino acid dimethyl ester is formed, and is then, after added with an alkylamine, further heated under reflux for amidation reaction, whereby the N-acylglutamic acid dialkylamide, for example, is formed.

Next, the purifying method of the present invention will be concretely described wherein the resultant, desired N-acylamino acid amide such as, e.g., N-acylglutamic acid dialkylamide, can be obtained in the pure state from a synthetic reaction mixture containing the said N-acylamino acid amide.

When subjected to the purifying method of the present invention, such synthetic reaction mixture can be used as it is. A residue can also be subjected to the purifying method of the present invention, which residue has resulted from distilling off the reaction solvent such as toluene or xylene or the alkylamine used as a starting material but remaining unreacted, under reduced pressure from the synthetic reaction mixture. These are each an example of a substance desired but being contaminated with impurities. In order for impurities to be removed more effectively acccording to the purifying method of the present invention, the alkylamine etc. are preferably removed to the utmost degree in advance by distilling under reduced presure.

First, the step of extracting impurities with the use of an acid (referred to hereinafter as "acid-extraction step") will be described, which can be carried out according to the present invention. This step is a manipulation wherein an N-acylamino acid amide product desired but being accompanied by impurities is heat-dissolved in an organic solvent, and the resulting solution is added and mixed with an aqueous acid solution and allowed to stand into two separate layers, whereby the desired substance has moved into the organic solvent layer, while the impurities, into the aqueous layer.

The pH value of an aqueous acid solution to be used in this step is preferably within a range where the main impurity alkylamine can be transformed into its acid addition salt in a substantial amount, in order for the contaminant alkylamine to be removed more effectively, and can be such that the aqueous layer shows a pH of, e.g., not more than 3, which aqueous layer has resulted from layer separation after a desired but contaminated substance after added and mixed with an organic solvent and an aqueous acid solution, is allowed to stand. As acids to be used to prepare aqueous acid solutions, there can be mentioned various inorganic or organic acids. Commercially preferred are inorganic acids such as hydrochloric acid, sulfuric acid, phosphoric acid, and nitric acid, and the most preferable are hydrochloric acid and sulfuric acid. Such aqueous acid solution can be prepared before it is added to the extraction vessel containing a desired but contaminated substance therein. Such solution can be also prepared in situ in the extraction vessel containing a desired but contaminated substance therein, by adding water and an acid thereto. In short, formation of an aqueous acid solution will do, which solution can take impurities out from a desired substance contaminated with the impurities.

As the organic solvent to be used in this step, those organic acids can be mentioned which are almost immiscible with water and can dissolve a desired N-acylamino acid amide. In case where the azeotropic distillation step explained hereinafter is employed together with the acid-extraction step explained just above, those organic solvents which can be used also for such azeotropic distillation step, are preferred with a view to simplifying the factory facilities conerned. As such organic solvents, there may be mentioned toluene, benzene, ethyl acetate, butyl acetate, xylene, cyclohexane, methyl ethyl ketone, methyl isobutyl ketone, cyclohexanone, carbon tetrachloride, dichloromethane, tetrachloroethylene, butanol, pentanol, hexanol, octanol, heptanol, cyclohexanol, benzyl alcohol and the like. Of these, toluene, ethyl ecetate, butyl acetate, xylene and cyclohexane are preferable, and toluene is the most preferable. Alcohols such as butanol are difficult to remove from a desired substance in the solid state and may provide rank odors to the desired substance, whereas those organic solvents which may be toxic, such as benzene and halomethanes, e.g., carbon tetrachloride and dichloromethane, may raise envioronmental problems. Toluene is inexpensive, of which the azeotropic temperature with water is about 85°C, and is therefore suitable for the azeotropic distillation step according to the present invention. The above-mentioned organic solution may of course be used singly, and in the form of a mixture of two or more thereof unless the effects of the present invention are prevented from being brought about.

The amount of an aqueous acid solution and that of an organic solvent (or the ratio thereof) are not particularly critical. The organic solvent is used in an amount required to form an organic solvent layer which can dissolve the desired substance fully therein. The aqueous acid solution is used in an amount not particularly critical. If, however, the resultant aqueous layer is in too small an amount, the extraction efficiency of the impurities into the aqueous layer may be reduced, which may, in turn, need plural extraction operations, whereas if in too large an amount, liquid waste may result in a larger amount. They can, in general, be used in amounts giving a volume ratio of 1/2-2, and may of course be used outside the range insofar as the effects of the present invention can be brought about.

Temperatures where the acid-extraction step should be carried out (referred to hereinafter as "acid-extraction temperature" ) depend upon an organic solvent to be used, and are preferably those temperatures lower than the temperature where the organic solvent distills azeotropically with water (referred to hereinafter as "azeotropic temperature" ). Even at temperatures higher than the azeotropic temperature, such step can be carried out, if measures of adding further organic acid or the like are taken to prevent the N-acylamino acid amide from being precipitated due to the concentrating. Such measures, however, make complicated the manipalations involved in the step, and therefore are not desirable. On the other hand, at too low temperatures, the organic solvent may be gelatinized by the desired substance N-acylamino acid amide, and therefore the acid-extraction step needs carrying out at temperatures high enough to prevent the gelatinization of the solvent. In the case of toluene taken as an example of the organic solvent, the preferred temperatures are within a range of 75-84°C. An organic solvent and water to be used for such acid extraction may be heated to an appropriate temperature after they have been added to the extraction vessel, or may be added thereto after in advance heated to such a temperature.

As other conditions, there may be mentioned enough mixing, e.g., by stirring, of the aqueous acid solution and the organic solvent solution having the desired substance dissolved therein. Time of such mixing, e.g., by stirring, varies depending upon the stirring conditions employed, and is, though not particularly critical, preferably 10 or more minutes. After such mixing, the resultant mixture is allowed to stand till layer separation has occurred, and the resultant aqueous layer is removed. Time for such mixture to be allowed to stand is a period required for the two (i.e., aqueous and organic) layers to be fully separated, and is usually 20 or more minutes.

By the above-described acid-extraction step, most of the alkylamine as the main impurity is removed into the aqueous layer. Such acid-extraction step may be repeated plural times, depending upon the efficiency of such removal.

Next, the step of extracting impurities with the use of an alkali (referred to hereinafter as "alkali-extraction step") will be described, which can be carried out according to the present invention. This step is the same step as the acid-extraction step explained above, except that an aqueous alkali solution is used in place of an aqueous acid solution, whereby the impurities are moved into the aqueous and middle layers.

As has been explained above, most of the alkylamine as the main impuirty is removed by the above-described acid-extraction step. Repeated acid-extraction can, however, only decrease the alkylamine moiety to a certain low concentration. It is, however, difficult to remove the same any more. Furthermore, the coloring matters which may cause a problem of coloring the end product N-acylamino acid amide, are difficult to remove by the acid-extraction step alone. In accordance with the present invention, the alkali-extraction step as will be described just below, if carried out in addition to the acid-extraction step described above, can reduce the alkylamine and the like in concentration to a lower level, and also remove the coloring matters effectively.

In carrying out such alkali-extraction step, an aqueous alkali solution and an organic solvent solution containing the desired substance are, after stirred, allowed to stand for some time, whereby the mixture separates into three layers, i.e., lower layer (aqueous layer), middle layer (strongly colored emulsion layer) and upper layer (organic solvent layer). At this stage, the aqueous and middle layers may be removed, but such removal may reduce the final recovery yield of the desired substance. Whereas, if the mass is allowed to stand for further time, the middle layer gets reduced in volume. If both the layers are removed when no decrement has been seen in the volume of the middle layer, the desired substance can be recovered without prejudice to its recovery yield, while both the alkylamine and the coloring matters may be removed to a further lower level. Impurities cannot be removed sufficiently by the removal of the aqueous layer alone.

The aqueous alkali solution is preferably 11 or more in pH value. At pH valves lower than 11, the organic solvent layer may get difficult to separate from the other layers. As the alkali to be used in preparing an aqueous alkali solution, there may be mentioned sodium hydroxide, potassium hydroxide and ammonia, and of these, sodium hydroxide is preferred. Such aqueous alkali solution can be prepared before it is added to the extraction vessel or can be prepared in situ in the extraction vessel by adding thereto water and an alkali, in the similar manner as the aqueous acid solution is prepared.

As the organic solvents to be used in this alkali-extraction step, there may be mentioned the same organic solvents as those to be used in the above-described acid-extraction step.

Temperatures where the alkali-extraction step can be carried out, can also be the same as those where the acid-extraction step can be carried out.

The amount of an aqueous alkali solution and that of an organic solvent (or the ratio thereof) can be the same as in the acid-extraction step.

In carrying out such alkali extraction step, an aqueous alkali solution and an organic solvent solution containing the desired substance need mixing sufficiently, e.g., by stirring, as in the acid-extraction step. Time of such mixing, e.g., by stirring, is not particuarly critical, and preferably 10 or more minutes. After such mixing, the resultant mixture is allowed to stand, whereby layer separation occurrs. In carrying out such alkali extraction step, sodium chloride can be, as usual, added to the aqueous layer, whereby layer separation can be facilitated. The middle layer is preferably reduced enough in its volume, as has been described earlier, and for this reason, time for such mixture to be allowed to stand can be 8 or more hours.

Such alkali extraction step following the acid extraction step described above can give an N-acylamino acid amide product of higher purity.

The acid-extraction step and the alkali-extraction step can be carried out each singly or in combination and once or plural times, in compliance with such qualities as purity required of an N-acylamino acid amide product. In order for the impurities to be removed most effectively, it is preferred to remove first most of the alkylamine by the acid extraction, then followed by the alkali extraction. If the alkali extraction is carried out at a high alkylamine concentration, the impurities may be removed less effectively.

Other than the acid extraction and the alkali extraction, there may be mentiond, extraction with the mere use of water and an organic solvent. The impurities may be removed remarkably less effectively by such extraction (See Example 2 given later).

Next, the azeotropic distillation step in accordance with the present invention, will be described.

The azeotropic distillation step is for obtaining an N-acylamino acid amide product in the form of a solid excellent in separability such as filterability. From crystallization by cooling or concentrating with the use of an organic solvent only, an N-acylamino acid amide is usually obtained in the form of fibrous crystals difficult to separate by filtering, such fibrous crystals being, in turn, solid remarkably unfavorable for commercial production. Whereas, an N-acylamino acid amide gets precipitated in the form of granular solid excellent in separability, by subjecting an organic solvent solution having the same dissolved therein together with water, and with further water being added if required, to azeotropic distillation in accordance with the present invention

As the organic solvent which may be used for the azeotropic distillation step ( "azeotropic solvent" ), there may be mentioned any organic solvent which is either sparingly miscible, or easily miscible, with wather, insofar as it can dissolve an N-acylamino acid amide and distill azeotropically with water. Examples of such organic solvent include cyclohexane, benzene, toluene, xylene, methanol, ethanol, n-propanol, isopropanol, n-butanol, 2-butanol, 2-pentanol, 1-hexanol, cyclohexanol, 1-octanol, 2-octanol, heptanol, benzyl alcohol, cyclohexanone, acetic acid, ethyl acetate, butyl acetate, acetone, methyl ethyl ketone, methyl isobutyl ketone, dioxane, chloroform, dichloromethane, tetrachloroethylene, and carbon tetrachloride. When the azeotropic distillation step is carried out in combination with the acid-extraction step and/or alkali-extraction step, it is preferable to use the same organic solvent as has been used in the extraction step(s), from the view point of using the organic solvent efficiently by recovering and reusing, simplifying the factory facilities, and the like. Such organic solvents may be singly or as a mixture of two or more thereof unless the effects of the present invention are prevented from being brought about.

When the acid-extraction and alkali-extraction steps are omitted, an reaction mixture or a residue thereof containing the desired substance as well as impurities is, after added with an organic solvent and water, directly subjected to the azeotropic distillation step. When such reaction mixture or a residue thereof has been treated by the extraction step(s), the resultant organic solvent layer may, after added with water, be subjected to the azeotropic distillation step.

Temperatures where the azeotropic distillation step should be carried out vary depending upon the organic solvent to be used. In case of toluene, the azeotropic temperature is about 85°C.

In the azeotropic distillation step, the desired N-acylamino acid amide gets precipitated as solid, as the solvent gradually changes from the organic solvent (e.g., toluene) to water which is a poor solvent for the desired substance, resulting from the azeotropic distillation of the organic solvent with water. In this way, an N-acylamino acid amide product can be obtained as large solid granules which are easy to separate, e.g., by filtering, whereas an N-acylamino acid amide product obtained in the conventional way is remarkably difficult to separate commercially.

As the crude N-acylamino acid amide product to be subjected to the azeotropic distillation step for purification, in accordance with the present invention, there may be mentioned a synthetic reaction mixture of an N-acylamino acid amide (contaminated with the reaction solvent such as methanol, and the starting material such as butylamine, used but remaining unreacted), a residue thereof resulting from removing the solvent by distillating (said residue being solid, and remaining contaminated with butylamine and the like which have not been removed by such solvent distillation), a solution of an N-acylamino acid amide in an organic solvent which has been subjected to the acid extraction and/or alkali extraction, a residue resulting from removing the solvent from such solution by distillating, and the like. If a solid of higher purity is wanted, the above-described acid extraction and/or alkali extraction should be carried out beforehand. It is not very effective to wash a once separated solid with the use of an aqueous acid solution and/or an aqueous alkali solution, because it is difficult to remove the impurities included in such solid.

To add a little in connection with the practice of the azeotropic distillation, it should be carried out with stirring. Without stirring, lumps would apt to be formed or scaling might be caused on the inside wall of the vessel. The pH of the aqueous layer is preferably from acidic to neutral, because solid granules when precipitated under alkaline conditions might get smaller in particle size. As the azeotropic distillation operation proceeds, the above-mentioned mixture usually gets slurried before the organic solvent is completely removed. Solid-liquid separation may be carried out at this stage, in order to separate the desired substance in the form of solid granules. Of course, however, such separation is preferably carried out after the organic solvent is completely removed, whereby the desired substance is recovered in higher yields. Furthermore, if the water is too much decreased in amount in the course of the azeotropic distillation, lumps are apt to be formed or scaling may be caused on the inside wall of the vessel. Therefore, the azeotropic distillation operation is preferably carried out, with the water being kept at an appropiate level by adding further water if necessary. Water can be of course allowed to be present at the outset in an amount sufficient enough to remove the organic solvent completely by the azeotropic distillation with water.

Finally, N-acylamino acid amides to which the acid-extraction step, the alkali-extraction step and the azeotropic distillation step, all described above, are to be applied, will be described.

The acyl moiety can be any acyl group insofar as it is a straight-chain or branched-chain, saturated or unsaturated, and aliphatic, aromatic or alicyclic acyl group having 1-30 carbon atoms. Examples of such acyl group include caproyl, capriroyl, lauroyl, myristoyl and stearoyl groups, or a mixture of two or more thereof.

The amide moiety can be derived from a straight-chain or branched-chain, saturated or unsaturatied, primary or secondary amine, or mono- or di-alcoholamine (e.g., monoethanolamine and diethanolamine), an aromatic amine, or an alicyclic amine, each having 1-60 carbon atoms, or ammonia. Examples of such amine include butylamene, octylamine, laurylamine, isostearylamine, stearylamine, benzylamine, and cyclohexylamine.

The amino acid from which the amino acid moiety can be derived, can be any of acidic, neutral and basic amino acids, or any of α-, β- and ε-amino acids. Examples of such amino acid include glycine, β-alanine, α-alanine, valine, serine, methionine, phenylalanine, 3,4-dioxyphenylalnine, aspartic acid, glutamic acid, lysine, ornithine, arginine, histidine, ε-aminocaproic acid and the like.

### (Best Mode for Carrying Out the Invention)

The present invention will be further illustrated with reference to Examples.

### Determination of n-Butylamine:

In the following Synthesis Example and Examples, n-butylamine accompanying, as an impurity, an N-acylamino acid butylamide product has quantatively been analyzed by the capillary electrophoresis. That is, 1 g precisely weighed of an N-acylamino acid bytylamide is added to 10 ml of ion-exchaned water. The mixture is subjected to dispersion treatment with the use of ultrasonic wave, followed by filtering. The filtrate is analyzed with the use of Hewlett-Packard Co.' capillary electrophoresis apparatus (3DCE, column : Barefused Silica 64.5cm × 50µm, Buffer pH3, Voltage 30kV). Quantative detection limit is 1 ppm.

### Synthesis Example 1 (Synthesis of N-acyl-L-glutamic acid dibutylamide)

Into a three-necked 3L flask equipped with a stirrer, a refluxing apparatus, a distillating apparatus and a dropping apparatus were added carefully a mixed solution of 66 g of conc. sulfuric acid and 1,163 g of methyl alcohol and 300.7 g of "Amisoft LS-11" (N-lauroyl-L-glutamic acid sodium salt, ex Ajinomoto Co., Inc.). The mixture was heated to 60-70°C and kept at the same temperature under reflux for 2 hours, whereby N-lauroyl-L-glutamic acid dimethyl ester was formed. The mass was added with 630 g (8.50 moles) of n-butylamine and kept at about 80°C under reflux for 4 hours.

The solvent was removed from the reaction mixture, by reduced pressure distillating, whereby a residue containing the desired substance N-lauroyl-L-glutamic acid di-n-butylamide was obtained (about 310 g).

### Comparative Example 1 (Crystallization from methanol)

The residue obtained in Synthesis Example 1 and containing N-lauroyl-L-glutamic acid di-n-butylamide was added with 3,500 g of methanol and heated to 50°C. The mixture was adequately stirred at this temperature, and the floating material was filtered off at the same temperature. The filtrate was cooled from 55°C to 30°C at a rate of 5°C per one minute.

The resulting fine fibrous crystals were separated at 30°C with the use of a centrifuge. The crystals were very bad in separability from the mother liquor because of its fibrous crystalline form, and released a strong rank odor of the amine (butylamine) when separated.

The crystals were taken out from the centrifuge and dried under reduced pressure, whereby 175.9 g (47.1% yield) of N-lauroyl-L-glutamic acid di-n-butylamide was obtained. The crystals after dried under reduced pressure were considerably reddish. The butylamine content remaining in the dried crystals was 3,200ppm.

### Example 1 (Toluene azeotropic distillation only)

A residue obtained as in Synthesis Example 1 was added with 680 g of toluene and heat-dissolved therein, and then with 600 g of water. Azeotropic distillation operation was carried out with further water being added from time to time in such way that the total volume of the water and the toluene was maintained at a level of the original volume of the first-added water and the toluene throughout the azeotropic distillation. As the toluene got removed (i.e., solvent replacement of toluene with water proceeded) in the course of the azeotropic distillation the N-lauroyl-L-glutamic acid di-n-butylamide got precipitated. After the toluene had been completely removed, a non-definitely shaped solid in the form of rice grains was separated with the use of a centrifuge. The solid was excellent in separability. However, the amine odor was strong when the solid was centrifuged, and the solid separated by centrifuging contained water in an amount of as much as about 60%, and was bad in driability (i.e., difficult to dry) in subsequently drying under reduced pressure. 311.6 g (83.4% yield) of solid N-lauroyl-L-glutamic acid di-n-butylamide was obtained by drying under reduced pressure. The solid was considerably reddish, and the butylamine content remaining in the solid was 3,000 ppm.

### Example 2 (Hot-water extraction and toluene azeotropic distillation combined)

Another redidue obtained as in Synthesis Example 1 was added with 680 g toluene and heat-dissolved therein, and then with 600 g of water. After the mixture had been stirred at 80°C for 15 minutes, it was allowed to stand at the same temperature for one hour. The resulting lower aqueous layer was removed. This hot-water extraction operation was repeated two more times, followed by the same azeotropic distillation as in Example 1, with water being added from time to time. As the toluene got removed in the course of the azeotropic distillation, granular solid got precipitated.

After the toluene had been completely removed, a non-definitely shaped solid in the form of rice grains was separated with the use of a centrifuge. The granular solid was easy to separate (excellent separability). However, the amine odor was strong, and the solid separated was bad in driability in subsequently drying under reduced pressure. 309.2 g (82.7% yield) of N-lauroyl-L-glutamic acid di-n-butylamide was obtained by drying under reduced presssure. The solid after dried was considerably reddish, and the butylamine content remaining in the solid was 3,000 ppm.

### Example 3 (Acid-extraction and toluene azeotropic distillation combined)

Still another residue obtained as in Synthesis Example 1 was added with 680 g of toluene and heat-dissolved therein, and then with 600 g of water. Further, about 330 g of 9N (normal) sulfuric acid was carefully added thereto. The mixture was stirred at 80°C for 15 minutes and then allowed to stand at the same temperature for 30 minutes. After it had been comfirmed that the pH value of the aqueous layer was less than 3; it was removed ( "acid-extraction" ). This sulfuric acid extraction operation was repeated two more times. The toluene layer left was added with 600 g of water and subjected to the same azeotropic distillation, as in Example 1, with water being added from time to time. As the toluene got removed in the course of the azeotropic distillation, granular solid got precipitated.

After the toluene had been completely removed, a non-definitely shaped solid in the form of rice grains thus precipitated was separated with the use of a centrifuge. The solid was excellent in separability, and gave almost no butylamine odor when centrifuged. The solid separated by centrifuging contained water in an amount of as much as about 70%, and was difficult to subsequently dry under reduced pressure (bad driability). The solid separated was dried under reduced pressure, whereby 312.2 g (84.0% yield) of N-lauroyl-L-glutamic acid di-n-butylamide was obtained. The solid after dried was slightly reddish. The butylamine content remaining in the solid was, when determined, 40 ppm.

### Example 4 (Acid extraction, alkali extraction and toluene azeotropic distillation combined)

A residue obtained as in Synthesis Example 1 was added with 680 g of toluene and heat-dissolved therein, and then with 600 g of water. Further, about 330 g of 9N sulfuric acid was carefully added thereto. The mixture was stirred at 80°C for 15 minutes and the allowed to stand at the same temperature for 30 minutes. After it had been comfirmed that the pH of the aqueous layer was below 3, it was discarded. This acid extraction operation was repeated two more times.

Next, 720 g of water, 25 g of sodium hydroxide, and 30 g of sodimu chloride was added to the remainder. The mixture was stirred at 80°C for 15 minutes, and allowed to stand at the same temperature for 12 hours. Out of the three resulting layers, only the aqueous layer (lower layer) was discarded. This alkali extraction operation was repeated once more. The toluene layer left was added with 660 g of water and further with dilute sulfuric acid in such amount that the lower layer (the aqueous layer) got to 7 in pH value. Azeotropic distillation was carried out, as in Example 1, with water being added from time to time. As the toluene got removed in the course of the azeotropic distillation, granular solid got precipitated.

After the toluene had been completely removed, a non-definitely shaped solid in the form of rice grains was separated with the use of a centrifuge. The solid was excellent in separability, and gave almost no amine odor when centrifuged. The solid separated by centrifuging contained water in an amount of about 35%. The solid was dried under reduced pressure, whereby 323.6 g (86.6% yield) of N-lauroyl-L-glutamic acid di-n-butylamide was obtained. The solid was good in driability and slightly reddish after dried. The butylamine content remaining in the solid was, when determined, 30 ppm.

### Example 5 (Acid extraction, alkali extraction and xylene azeotropic distillation combined)

A residue obtained as in Synthesis Example 1 was added with 680 g of xylene and heat-dissolved therein, and then with 600 g of water. Further, about 330 g of 9N sulfuric acid was carefully added thereto. The mixture was stirred at 80°C for 15 minutes, and then allowed to stand at the same temperature for 30 minutes. After it had been confirmed that the pH value of the aqueous layer was less than 3, it was discarded. This sulfuric acid extraction operation was repeated two more times.

Next, 720 g of water, 25 g of sodium hydroxide, and 30 g of sodium chloride was added to the remainder. The mixture was stirred at 80°C for 15 minutes, and allowed to stand at the same temperature for 12 hours. Out of the three resulting layers, only the aqueous layer (lower layer) was discarded. This alkali extraction operation was repeated once more. The xylene layer left was added with 660 g of water and further with dilute sulfuric acid in such amount that the lower layer (the aqueous layer) got to 7 in pH value. Azeotropic distillation was carried out, as in Example 1 with water being added from time to time. As the xylene got removed in the course of the azeotropic distillation, granular solid got precipitated.

After the xylene had been completely removed, a non-definitely shaped solid in the form of rice grains was separated with the use of a centrifuge. The solid was excellent in separability, and gave almost no amine odor when centrifuged. The solid separated by centrifuging contained water in an amount of about 35%. The solid was dried under reduced pressure, whereby 318 g (85.1% yield) of N-lauroyl-L-glutamic acid di-n-butylamide was obtained. The solid was good in driability and slightly reddish after dried. The butylamine content remaining in the solid was, when determined, 40 ppm.

### Example 6 (Acid extraction, alkali extraction and toluene azeotropic distillation combined)

A residue obtained as in Synthesis Example 1 was added with 680 g of toluene and heat-dissolved therein, and then with 600 g of water. Further, about 330 g of 9N sulfuric acid was carefully added thereto. The mixture was stirred at 80°C for 15 minutes and then allowed to stand at the same temperature for 30 minutes. After it had been confirmed that the pH of the aqueous layer was below 3, it was discarded. This acid extraction operation was repeated two more times.

Next, 720 g of water, 25 g of sodium hydroxide, and 30 g of sodium chloride was added to the remainder. The mixture was stirred at 80°C for 15 minutes, and allowed to stand at the same temperature for 12 hours. Out of the three resulting layers, the aqueous layer was discarded, and then the middle layer was dicarded too. This alkali extraction operetion was repeated once more.

The toluene layer left was added with 660 g of water and further with dilute sulfuric acid in such amount that the lower layer (the aqueous layer) got to 7 in pH value. Azeotropic distillation was carried out, as in Example 1, with water being added from time to time. As the toluene got removed in the course of the azeotropic distillation, granular solid got precipitated.

After the toluene had been completely removed, a non-definitely shaped solid in the form of rice grains was separated with the use of a centrifuge. The solid was excellent in separability, and gave no amine odor when centrifuged. The solid separated by centrifuging contained water in an amount of about 35%, and was good in driability in subsequently drying under reduced pressure. The solid separated was dried under reduced pressure, whereby 299.1 g (80.0% yield) of N-lauroyl-L-glutamic acid di-n-butylamide was obtained. The solid was white after dried. The butylamine content remaining in the solid was, when determined, less than 1 ppm.

### (Industrial Applicability)

In accordance with the purifying method of the present invention, an N-acylamino acid amide can be commercially easily separated in the form of solid excellent in separability, e.g., by filtering, and, in turn, of higher purity.

## Claims

1. A method of purifying an N-acylamino acid amide which comprises having a solution of an N-acylamino acid amide in an organic solvent and water exist together, distillating the organic solvent and the water azeotropically, whereby the N-acylamino acid amide is precipitated as solid, and separating the solid.

2. A method of purifying an N-acylamino acid amide which comprises carrying out once or more times at least one extraction operation selected from the group consisting of the acid extraction carried out by having an organic solvent solution of an N-acylamino acid amide contaminated with impurities such as the alkylamine used as a starting material but remaining unreacted and an aqueous acid solution exist together, and the alkali extraction carried out by having the said organic solvent solution and an aqueous alkali solution exist together.

3. A method of purifying an N-acylamino acid amide which comprises carrying out at least once the acid extraction carried out by having an organic solvent solution of an N-acylamino acid amide contaminated with impurities such as the alkylamine used as a starting meterial but remainging unreacted and an aqueous acid solution exist together, and the alkali extraction carried out by having the said organic solvent solution and an aqueous alkali solution exist together, in this order.

4. The method of purifying an N-acylamino acid amide as set forth in Claim 1, which further comprises carrying out at least once the said acid extraction and/or the said alkali extraction, prior to the azeotropic distillation.

5. The method of purifying an N-acylamino acid amide as set forth in Claim 4, which further comprises carrying out at least once the said acid extraction and the said alkali extraction in this order, prior to the azeotropic distillation.

6. The method of purifyting an N-acylamino acid amide as set forth in Claim 1, 4 or 5, wherein the organic solvent to be used for the azeotropic distillation is one member selected from the group consisting of cyclohexane, benzene, toluene, xylene, methanol, ethanol, n-propanol, isopropanol, n-butanol, 2-butanol, 2-pentanol, 1-hexanol, cyclohexanol, 1-octanol, 2-octanol, heptanol, benzyl alcohol, cyclohexanone, acetic acid, ethyl acetate, butyl acetate, acetone, methyl ethyl ketone, methyl isobutyl ketone, dioxane, chloroform, dichloromethane, tetrachloroethylene and carbon tetrachloride or a mixture of two or more thereof.

7. The mixture of purifying an N-acylamino acid amide as set forth in any one of Claims 2-5, wherein the organic solvent to be used for the said acid extraction or the said alkali extraction is one member selected from the group consisting of cyclohexane, toluene, benzene, xylene, methyl ethyl ketone, methyl isobutyl ketone, cyclohexanone, butyl acetate, carbon tetrachloride, dichloromethane, tetrachloroethylene, butanol, pentanol, hexanol, octanol, heptanol, cyclohexanol and benzyl alcohol or a mixture of two or more thereof.

8. The method of purifying an N-acylamino acid amide as set forth in any one of Claims 1-5, wherein the said organic solvent to be used for the azeotropic distillation, the acid extraction or the alkali extraction is toluene or xylene.
